(19)

Europäisches
Patentamt

European
Patent Office

Office européen
des brevets

(11) EP 3 400 984 A1

(12) EUROPEAN PATENT APPLICATION

(43) Date of publication:
14.11.2018 Bulletin 2018/46

(51) Int Cl.:
A61M 16/00 (2006.01)     A61M 16/12 (2006.01)

(21) Application number: 17290059.9

(22) Date of filing: 08.05.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Goutorbe, Philippe
83100 Toulon (FR)

(72) Inventor: Goutorbe, Philippe
83100 Toulon (FR)

(74) Representative: Appleyard Lees IP LLP
15 Clare Road
Halifax HX1 2HY (GB)

(54) SYSTEMS AND METHODS FOR AUTOMATICALLY ADJUSTING A DETERMINED SUPPLY OF FIO2 GENERATED FROM A CPAP, NIV OR OTHER VENTILATOR SYSTEM

(57)     A medical ventilator system configured for use by an individual at home, the ventilator system including: an oxygen source; an air flow generator; an oxygen supply control valve; a patient supply tube; a pressure sensor; one or more flow sensors; a controller; wherein the airflow generator combines the amount of oxygen and the amount of ambient air to form breathing air, wherein the breathing air is supplied to the patient at a pressure above atmospheric pressure; and wherein the controller is operable to control a flow rate and pressure of the breathing air from the air flow generator and wherein the controller is operable to control an oxygen flow rate to the air flow generator from the oxygen source by selectively opening, closing, or throttling the oxygen control valve.

FIG. 1

**Description**

COPYRIGHT INFORMATION

A portion of the disclosure of this patent document contains material that is subject to copyright protection. The copyright owner has no objection to the facsimile reproduction by anyone of the patent document or the patent disclosure, as it appears in the Patent and Trademark Office patent file or records, but otherwise reserves all copyright rights whatsoever.

FIELD OF THE INVENTION

The present invention relates to ventilators, as well as continuous positive airway pressure (CPAP) machines, and more particularly to such machines which provide supplemental oxygen to a user wherein the control of said such machines is varied in response to a sensed and targeted fraction of inspired oxygen.

BACKGROUND OF THE INVENTION

There exist today numerous maladies and treatments thereof that include the use of a ventilator or breathing assistance apparatus. Many systems which exist in the art today, typically include a venting means configured to compress ambient air, for example a turbine or a bellows, which sends ambient air optionally enriched with oxygen in a supply tube so as to supply this air to a connected mask being fixed about the patient's head, or to an intra-tracheal device allowing the patient to inhale the air-oxygen mixture.

Certain ventilators, CPAP machines and the like can be configured with a target airflow and/or pressure setting to be provided into a supply tube. In the case of leakage in a system, such as leaks at the mask, which might result from a mask being incorrectly positioned or sealed about the patient's head, ventilator and the like (NIV or CPAP) systems can detect said leakage and then alter the turbine or blower speed so as to maintain the set target pressure. The increased (or decreased) turbine or blower speed alters the amount of ambient air in the supply tube. This can be problematic because the rate at which oxygen (or other gas) is added to a ventilator is done through setting an O2 supply to flow at a set constant value, which is usually in the ratio of liters per minute. Thus, without adapting the amount of oxygen delivered into the supply tube, the change in ambient air supply causes an unwanted decrease or increase in the fraction of inspired oxygen (herein referred to as FiO2) that is being supplied.

A change in FiO2 can cause a large number of rapidly forming complications, such as an increase of pulmonary arterial hypertension (hypoxemia), which can result in great harm to, or even death of, the patient. Therefore, if a system cannot automatically adjust the O2 (or other gas) to correspond with an increase or decrease in ambient air intake results as a reaction to maintaining a target pressure, caused be leakage, problems can occur.

Additionally, present systems typically sense the patient blood oxygen levels using a sensor attached to directly to the patient, and adjust the oxygen input so as to increase or decrease the blood oxygen, such sensors can include SpO2(peripheral capillary oxygen saturation) sensors. However, due to the fact that it may take several breathing cycles before a patient's blood oxygen levels begin to fluctuate, using such sensors cause a significant amount of lag between when an adjustment to FiO2 should be made, and when the systems actually respond. Thus, a system is needed that can adjust FiO2 input and do so in a highly responsive manner.

SUMMARY OF THE INVENTION

In this context, the present invention aims to provide a medical ventilator for home ventilation that solves this problem in changes in FiO2 by varying both pressure of the air supply along with supplemental oxygen supply. Additionally, the system provided herein can provide changes to FiO2 input levels in a manner that is more responsive than SpO2 sensor-based systems. As such, the present invention as contemplated herein can include a medical ventilator intended for home use which includes a blower for receiving ambient air and providing it to a patient at an increased pressure, an oxygen source configured to supply oxygen at a controlled rate to the blower, and a calculating means configured to vary both the pressure and the oxygen supply rate so as to provide air to the patient having a target FiO2 in response to either a sensed or calculated FiO2 level in the supply tube.

In particular, the present invention includes medical ventilator system configured for use by an individual at home, the ventilator system having an oxygen source, and an air flow generator operatively connected to receive an amount of oxygen from the oxygen source along with an amount of ambient air.

Between the air flow generator and the oxygen source an oxygen control valve can be provided, the control valve being configured to modulate a rate of oxygen being provided to the air flow generator.

A patient supply tube can then be provided about an outlet of the air flow generator so as to receive an amount of ambient air and an amount of oxygen from the air flow generator above ambient pressure, being pressurized by the air flow generator, and deliver the amount of ambient air and the amount of oxygen to a patient interface provided at an opposing end of the patient supply tube. It will be appreciated that the patient interface can then be configured to interface with one or more breathing airways of a patient through the use of a mask assembly or a nasal pillow assembly, or any number of other airway or patient interfaces as will be appreciated by those having skill in the art.

In some embodiments a pressure sensor provided between the patient and the air flow generator. Additionally, in some embodiments a flow sensor provided between the oxygen source and the air flow generator.

A controller can then be provided in a manner so as to receive input from the pressure sensor and the flow sensor and can adjust the oxygen control valve and air flow generator output so as to maintain the air in the patient supply tube so as to have a desired or target FiO2. It will be appreciated that the oxygen control valve can be provided as a proportional solenoid valve in some embodiments. In yet additional embodiments the air flow generator can include a variable speed turbine or a compressor.

In yet additional embodiments the controller can also include an input module for receiving an input related to a fraction of inspired oxygen (FiO2) setting. This can be received as an input from the user or from a remotely run application which is then configured to send instructions or settings to the controller.

In some embodiments the controller can be configured to compare the FiO2 input having a target Fi02 with a calculated FiO2 value that is based on input data from both the pressure sensor and flow sensor.

Also contemplated herein is a method of providing supplemental respiration to a patient in a home environment, the method comprising the steps of: providing an oxygen source; providing an air flow generator operatively connected to receive an amount of oxygen from the oxygen source, the air flow generator also being configured to receive an amount of ambient air; providing an oxygen control valve being provided between the oxygen source and the air flow generator; providing a patient supply tube being configured to receive the amount of oxygen and the amount of ambient air from the air flow generator and deliver the amount of oxygen and the amount of ambient air to a patient interface as breathing air, i.e. a mixture of gas having ambient air and supplemental oxygen from the oxygen source, which is then supplied at a pressure above atmospheric pressure; providing a controller, the controller being configured to receive input from one or more sensors regarding characteristics of the breathing air; determining an actual fraction of inspired oxygen (Fi02) value within the breathing air being supplied to the patient; connecting the patient supply tube in an operative manner to one or more airways of the patient; receiving a target pressure by the controller; receiving a target fraction of inspired oxygen (Fi02) value for the breathing air by the controller; and increasing or decreasing the output of the air flow generator so as to maintain the target pressure; opening or throttling the oxygen control valve so as to maintain the actual fraction of oxygen in the breathing air at the target fraction of inspired oxygen (FiO2) value.

In some embodiments the method can further include the additional step of: providing an oxygen flow sensor between the oxygen source and the air flow generator; and wherein the determination of the actual fraction of inspired oxygen (FiO2) value is determined based on input received by the controller from the oxygen flow sensor.

In yet additional embodiments the method can further include the additional step of: providing a breathing air flow sensor on the patient supply tube; wherein the determination of the actual fraction of inspired oxygen is determined based on input received by the controller from the breathing air flow sensor.

In some embodiments, the method can further include the additional steps of: determining an output flow rate of the air flow generator based on a sensed turbine speed.

In some embodiments, the method can further include the additional steps of: recognizing a pressure change in the patient supply tube; varying an output of the air flow generator so as to compensate for the pressure change; and varying an oxygen flow from the oxygen source to compensate for the increased output of the air flow generator so as to maintain the target fraction of inspired oxygen.

Also contemplated herein is another embodiment of a method of providing supplemental respiration to a patient in a home environment, the method comprising the steps of: providing an oxygen source; providing an air flow generator operatively connected to receive an amount of oxygen from the oxygen source, the air flow generator also being configured to receive an amount of ambient air; providing an oxygen control valve being provided between the oxygen source and the air flow generator; providing a patient supply tube being configured to receive the amount of oxygen and the amount of oxygen from the air flow generator and deliver the amount of oxygen and the amount of oxygen to a patient interface; providing a controller, the controller being configured to receive input from one or more sensors regarding characteristics of the breathing air; wherein the airflow generator combines the amount of oxygen and the amount of ambient air to form a breathing air, wherein the breathing air is supplied to the patient at a pressure above atmospheric pressure; determining an actual fraction of inspired oxygen within the breathing air being supplied to the patient; connecting the patient supply tube in an operative manner to one or more airways of the patient; receiving a target pressure by the controller; receiving a target fraction of inspired oxygen value for the breathing air by the controller; opening or throttling the oxygen control valve so as to maintain the actual fraction of oxygen in the breathing air at the target fraction of inspired oxygen; recognizing a pressure change in pressure in the patient supply tube; varying an output of the air flow generator so as to compensate for the pressure change; varying an oxygen flow from the oxygen source to compensate for the increased output of the air flow generator so as to maintain the target fraction of inspired oxygen; providing an oxygen sensor on the patient supply tube; providing a pressure sensor on the patient supply tube; and wherein the determination of the actual fraction of inspired oxygen is based on input received from by the controller from the oxygen sensor and the pressure sensor.

In some embodiments this method can further include the additional steps of: sensing the flow of oxygen from the oxygen source prior to the oxygen being combined; determining an estimated fraction of inspired oxygen (FiO2) value by a controller based on input received from sensing both the flow of oxygen and flow or pressure of breathing air; comparing the estimated FiO2 value with a target FiO2 value; and adjusting an oxygen flow rate of the oxygen source for oxygen received by the airflow generator.

In some embodiments, this method can further include the additional steps of: adjusting oxygenated air for medicinal use, wherein determining the estimated FiO2 value is based on the Formula 1:

$$Estimated\ FiO2 = \frac{(Total\ flow - O2\ flow) * A + (O2 * B)}{Total\ flow}$$

Where A is defined as the percentage of oxygen content in the ambient air surrounding the patient, which can be either set by the user or identified by a sensor in the ventilator, typically this could be a barometric sensor, and where B is defined as the percentage of oxygen in the outlet flow of the supplemental oxygen source which can also be either set by the user or identified by a sensor.

In some embodiments, this method can further include the additional steps of: measuring the pressure of breathing air prior to the breathing air being received by the user interface; comparing the measured breathing air with a target breathing air pressure value; and adjusting the airflow generator based on the comparison. In some such embodiments, the determining the estimated FiO2 value step can be performed after the adjusting of the airflow generator.

BRIEF DESCRIPTION OF THE DRAWINGS

The foregoing and other objects, features, and advantages of the invention will be apparent from the following description of particular embodiments of the invention, as illustrated in the accompanying drawings in which like reference characters refer to the same parts throughout the different views. The drawings are not necessarily to scale, emphasis instead being placed upon illustrating the principles of the invention.

FIG. 1 illustrates a diagram of a ventilator system configured to implement various methods and aspects of the present invention

FIGs. 2A-E illustrates various tabulated scenarios illustrative of the method of controlling the FiO2 input provided by a CPAP or ventilator system.

DETAILED DESCRIPTION OF THE INVENTION

The ventilator system 10 is remarkable in that it comprises regulation means of varying the oxygen flow rate, i.e. a valve, being operatively connected to a controller. Additionally, various sensors are provided on the patient supply tube, one of which being configured to measure supply air pressure, and flow, and another being configured to measure oxygen supplied, so as to calculate or otherwise measure the fraction of inspired oxygen (Fi02). The controller can then utilize a processor so as to calculate the FiO2 and adjust the pressure by means of the gas flow generator as well as the oxygen supply rate so as to achieve a target FiO2 level.

The regulation of the supplied oxygen is achieved by the controller in response to the FiO2 determined in the air supply tube to the patient, and thus by measuring or otherwise calculating the FiO2 of the air being supplied to the patient. Advantageously, the air flow generator can include a regulating turbine or blower, the outlet pressure or flow rate of which can also be controlled by the controller based on the measurement of the FiO2.

It will be understood that regulatory turbines in gas flow generators can adjust speeds to quickly change so as to adjust to user breath intake and load requirements. It will be appreciated that in many treatment applications, such as for continuous positive air pressure supply for the treatment of sleep apnea, that a constant pressure is often desired for purposes of treatment. As such, leakage variations during user repositioning, or natural breathing movement can be accounted for fairly easily by rapidly increasing or decreasing turbine speed in response to a sensed pressure change within the patient supply tube 120. It will be further understood, that a corresponding increase or decrease of turbine speed, flow, pressure, etc. can have an effect of FiO2 and as such the oxygen supply rate can be adjusted so as to maintain a target FiO2.

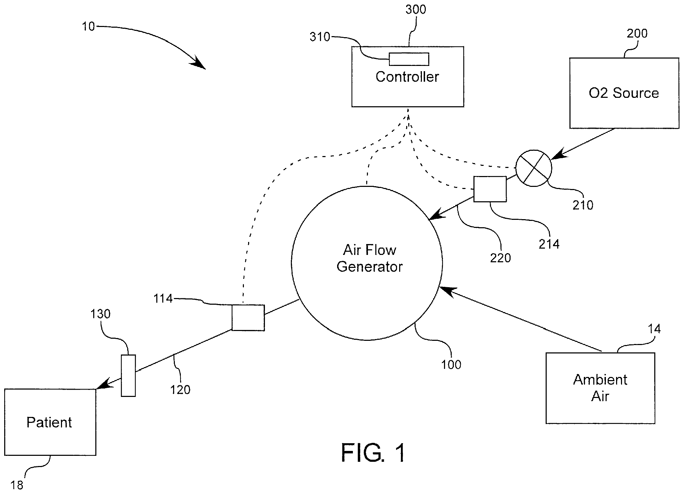

In order to achieve a regulated FiO2, the present invention as contemplated herein can include a medical ventilator and system 10, as illustrated in FIG. 1, wherein the system 10 is intended for home use.

The ventilator system 10 can include an air flow generator 100 which can be configured to receive ambient air 14 and provide it to a patient at an increased pressure, the ambient air can be supplemented with additional oxygen from an oxygen source 200.

The oxygen source 200 can be connected to a separate inlet of the air flow generator 100 such that the air flow generator 100 can receive both oxygen from the oxygen source and ambient air from the surroundings of the air flow generator, mix the two, and deliver the air to a patient through a patient supply tube having an increased pressure as well as an increased fraction of oxygen contained therein. It will be appreciated that the total fraction of oxygen in the patient supply tube can be referred to as a fraction of inspired oxygen, herein referred to as FiO2.

The oxygen source can then be configured to supply oxygen at a controlled rate to the blower or other air flow generator 100. This controlled rate of oxygen can be achieved by providing a controllable valve 210 on the oxygen supply line or tube and can be referred to herein as an oxygen control valve, and can be opened completely closed completely or incrementally throttled to any rate between maximum flow and zero flow by incrementally closing or opening the valve.

It will be appreciated that one or more sensors 214 can also be provided on the oxygen supply line 220. Such sensors can include flow rate sensors, oxygen saturation or purity sensors which can provide information regarding the purity or fraction of oxygen supplied from the oxygen source.

Additional sensors 114 can also be provided on the patient supply tube 120. Such sensors can also include pressure, turbidity, flow rate, etc.

The sensors 214 and 114 can be attached to a controller 300 which is configured to receive data regarding the flow of ambient air and oxygen into and out of the air flow generator 100. The controller 300 can then be configured such that it can vary the oxygen control valve 210 and a turbine speed, or other air compression means, of the air flow generator 100 so as to achieve a desired FiO2. As such, the controller 300 can include a calculating means configured to vary both the pressure and the oxygen supply rate so as to provide air to the patient having a target FiO2 in response to a sensed or calculated Fi02 level in the supply tube 220.

A patient supply tube 120 can then be provided about an outlet of the air flow generator 100 so as to receive an amount of ambient air and an amount of oxygen from the air flow generator above ambient pressure so as to form breathing air. The breathing air is pressurized by the air flow generator to a therapeutic level as determined by a health care professional. It will be appreciated that the patient interface can then be configured to interface with one or more breathing airways of a patient 18 through the patient interface, i.e. by using a mask assembly or a nasal pillow assembly, or any number of other airway or patient interfaces as will be appreciated by those having skill in the art.

As discussed above, one of the sensors provided on the patient supply tube 120 can include a pressure sensor. In this manner, the system 10 and particularly the controller 300 can then detect leaks between the air flow generator 100 and the patient 18. It will then be appreciated that leaks typically manifests themselves as a pressure drop within the patient supply tube 120. The air flow generator 100 can then be configured to increase the flow rate or pressure so as to compensate for the pressure drop thus increasing the flow of the air within the pressure supply tube 120. It will then be appreciated that merely increasing the flow rate by receiving and pressurizing additional ambient air will then cause an immediate drop in FiO2 without a corresponding increase in the oxygen supply rate. In order to aid in the speed of FiO2 of the breathing air, a flow sensor can be included in the one or more sensors 214 provided on the oxygen supply line 220. Then the oxygen flow rate can be adjusted either up or down by varying the oxygen control valve 210.

The controller can also be provided with an input module or other input means 310. The input means can be a touch screen or other input mechanism for receiving one or more parameters from the user. Such parameters can include a target pressure, which is common for treatment of certain maladies, including sleep apnea, where breathing air is provided at a certain level above atmospheric or ambient pressure. Such parameters will also include a target FiO2 as prescribed by a physician. It will be appreciated that the controller can also be configured to communicate over wireless or plug-in interfaces wherein the parameters can be input using applications provided on alternative devices such as smart phones, computer terminals, etc.

It will be appreciated that in some embodiments the oxygen control valve 210 can be provided as a proportional solenoid valve or any number of electronically controlled valves, including servo or any number of valves which will be readily apparent by those having skill in the art.

In yet additional embodiments the air flow generator can include a variable speed turbine, a compressor or any other air compression means which can provide breathing air in sufficient flow rates and at a desired temperature. However, it has been recognized that variable speed turbines can provide readily ascertainable and easily adjusted flow rates.

In some embodiments, the controller can be configured to compare the target Fi02 being input by the user with a calculated or otherwise determined actual FiO2 value which reflects the actual FiO2 being provided in the patient supply tube. This determination of actual FiO2 can be based on input data from any of the aforementioned sensors, including the pressure sensor in the patient supply tube, and flow sensor(s) on the oxygen supply line, and or the patient supply tube.

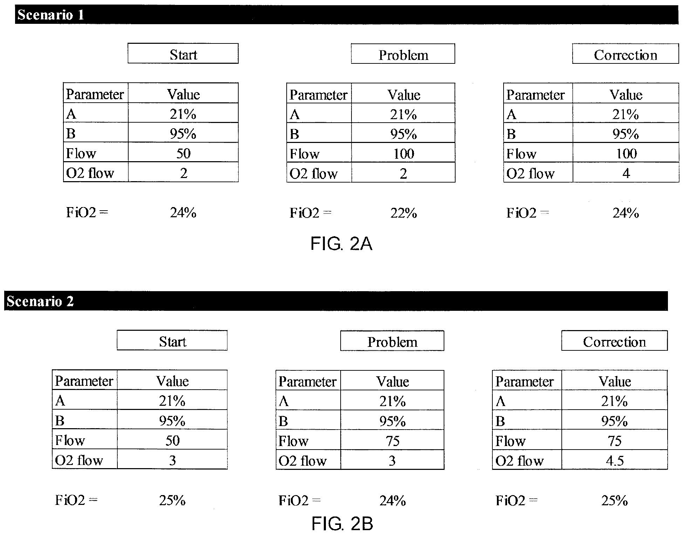

FIGs. 2A-E illustrates various tabulated scenarios illustrative of the method of controlling the ventilator system 10 wherein the ventilator system is utilized so as to provide supplemental respiration to a patient in a home environment, the method comprising the steps of: providing an oxygen source 200; providing an air flow generator 100 operatively connected to receive an amount of oxygen from the oxygen source 200, the air flow generator 100 being configured to receive an amount of ambient air; providing an oxygen control valve 210 being provided between the oxygen source and the air flow generator; providing a patient supply tube 120 being configured to receive the amount of oxygen and the amount of ambient air from the air flow generator and deliver the amount of oxygen and the amount of ambient air to a patient interface 130 as breathing air at a pressure above atmospheric pressure; providing a controller 300, the controller being configured to receive input from one or more sensors 114/214 regarding characteristics of the breathing air; determining an actual fraction of inspired oxygen (FiO2) value within the breathing air being supplied to the patient; connecting the patient supply tube 120 in an operative manner to one or more airways of the patient 18; receiving a target pressure by the controller; receiving a target fraction of inspired oxygen (Fi02) value for the breathing air by the controller; and increasing or decreasing the output of the air flow generator so as to maintain the target pressure; opening or throttling the oxygen control valve so as to maintain the actual fraction of oxygen in the breathing air at the target fraction of inspired oxygen (Fi02) value.

It will be appreciated that the input from the oxygen source can be controlled alternatively by controlling the oxygen supply source directly, such as throttling a motor, or otherwise affecting the supply rate of the supplemental oxygen.

These types of 114/214 sensors can include specifically: airflow sensors, pressure sensors, oxygen sensors, humidity sensors, air temperature sensors, barometric pressure sensors, and so forth. The type of sensor used can effectuate the responsiveness of the system. For example, if 114 contains both a pressure and an airflow sensor, the pressure sensor can be used by the system to detect leakage or other pressure drops in the system. This in turn causes a signal to be sent to a controller that would increase or decrease the turbine (air flow generator) speed to adjust. As such, the air flow rate, changes. Pressure sensors can be used as a part of a calculation to determine the airflow speed; however, pressure sensors and inherently pressure building up in a given system results in a lag of determining the actual airflow speed. Whereas, an airflow sensor can detect the airflow changes instantaneously and report those changes to a controller that adjusts oxygen (or other gas) flow rates to adjust in real-time, as discussed below in various embodiments.

In some embodiments contemplated herein volume and flow delivery in the ventilators or CPAP device can be calculated and displayed in terms of Body Temperature and Pressure Saturated (BTPS) and therefore humidity sensor and air temperature sensors can be used as input to the calculations in order to calculate the air flow into that domain (BTPS) giving the input to Formula 1.

$$\text{Estimated } FiO2 = \frac{(Total\ flow - O2\ flow)*A + (O2*B)}{Total\ flow}.$$ The barometric pressure sensor can be used to calculate the altitude so as to compensate for Oxygen saturation at the particular barometric pressure or altitude and thus compensate for the lower 02 content in surrounding air at higher altitudes and secondly; provide input to the pressure generator to compensate for the lower air mass flow and pressure at higher altitudes.

In some embodiments, the method can further include the additional step of: providing an oxygen flow sensor between the oxygen source and the air flow generator; and wherein the determination of the actual fraction of inspired oxygen (FiO2) value is determined based on input received by the controller from the oxygen flow sensor.

In yet additional embodiments, the method can further include the additional step of: providing a breathing air flow sensor on the patient supply tube; wherein the determination of the actual fraction of inspired oxygen is determined based on input received by the controller from the breathing air flow sensor.

In some embodiments, the method can further include the additional steps of: determining an output flow rate of the air flow generator based on a sensed turbine speed.

In some embodiments, the method can further include the additional steps of: recognizing a pressure change in the patient supply tube; varying an output of the air flow generator so as to compensate for the pressure change; and varying an oxygen flow from the oxygen source to compensate for the increased output of the air flow generator so as to maintain the target fraction of inspired oxygen.

Also contemplated herein is another embodiment of a method of providing supplemental respiration to a patient in a home environment, the method comprising the steps of: providing an oxygen source; providing an air flow generator operatively connected to receive an amount of oxygen from the oxygen source, the air flow generator also being configured to receive an amount of ambient air; providing an oxygen control valve being provided between the oxygen source and the air flow generator; providing a patient supply tube being configured to receive the amount of oxygen and the amount of oxygen from the air flow generator and deliver the amount of oxygen and the amount of oxygen to a patient interface;

providing a controller, the controller being configured to receive input from one or more sensors regarding characteristics of the breathing air; wherein the airflow generator combines the amount of oxygen and the amount of ambient air to form a breathing air, wherein the breathing air is supplied to the patient at a pressure above atmospheric pressure; determining an actual fraction of inspired oxygen within the breathing air being supplied to the patient; connecting the patient supply tube in an operative manner to one or more airways of the patient; receiving a target pressure by the controller; receiving a target fraction of inspired oxygen value for the breathing air by the controller; opening or throttling the oxygen control valve so as to maintain the actual fraction of oxygen in the breathing air at the target fraction of inspired oxygen; recognizing a pressure change in pressure in the patient supply tube; varying an output of the air flow generator so as to compensate for the pressure change; varying an oxygen flow from the oxygen source to compensate for the increased output of the air flow generator so as to maintain the target fraction of inspired oxygen; providing an oxygen sensor on the patient supply tube; providing a pressure sensor on the patient supply tube; and wherein the determination of the actual fraction of inspired oxygen is based on input received from by the controller from the oxygen sensor and the pressure sensor.

In some embodiments this method can further include the additional steps of: sensing the flow of oxygen from the oxygen source prior to the oxygen being combined; determining an estimated fraction of inspired oxygen (FiO2) value by a controller based on input received from sensing both the flow of oxygen and flow or pressure of breathing air; comparing the estimated FiO2 value with a target FiO2 value; and adjusting an oxygen flow rate of the oxygen source for oxygen received by the airflow generator.

In some embodiments, this method can further include the additional steps of: adjusting oxygenated air for medicinal use, wherein determining the estimated FiO2 value is based on Formula 1:

$$Estimated\ FiO2 = \frac{(Total\ flow - O2\ flow) * A + (O2 * B)}{Total\ flow}$$

Where A is defined as the percentage of oxygen content in the ambient air surrounding the patient, which can be either set by the user or identified by a sensor in the ventilator, typically this could be a barometric sensor, and where B is defined as the percentage of oxygen in the outlet flow of the supplemental oxygen source which can also be either set by the user or identified by a sensor.

In some embodiments, this method can further include the additional steps of: measuring the pressure of breathing air prior to the breathing air being received by the user interface; comparing the measured breathing air with a target breathing air pressure value; and adjusting the airflow generator based on the comparison. In some such embodiments in the determining the estimated FiO2 value step can be performed after the adjusting of the airflow generator.

Contemplated herein is a method of treatment using a device as described above so as to provide a target FiO2 so as to keep the patient in therapeutic zone, for example 28 to 32%.

The goal of the method is to adjust the flow of 02 as a function of the air flow of the air flow generator. By providing a proportional valve downstream of an 02 supply source, such as a compressed 02 cylinder or another similar 02 source, the flow of 02 can be modulated.

In one embodiment, the controller can include a microprocessor and associated control software which operates the system so as to provide a constant FiO2 regardless of a particular pressure or flow generated by the air flow generator. The target Fi02 can be modulated in accordance with Formula 1: $Estimated\ FiO2 = \frac{(Total\ flow - O2\ flow)*A+(O2*B)}{Total\ flow}$. Again, where A is defined as the percentage of oxygen content in the ambient air surrounding the patient, which can be either set by the user or identified by a sensor in the ventilator, typically this could be a barometric sensor, and where B is defined as the percentage of oxygen in the outlet flow of the supplemental oxygen source which can also be either set by the user or identified by a sensor.

Where total flow is defined as the gas flow leaving the air outlet of the air flow generator during the inspiratory and expiratory phase which can either be identified from the air flow generator based on turbine speed, or from a sensor external from the air flow generator, such as a flow meter. It will also be appreciated that flow rates can also be calculated based on a plurality of pressure sensors or pitot tubes, as will be readily apparent to those having skill in the art of flow measurement and detection.

As discussed above, FIGs. 2A-E illustrates tables of various scenarios illustrative of the implementation of the aforementioned methods.

As such FIG. 2A illustrates a first scenario in which a leak, such as around the mouth of a mask or other increased leak around the mask is detected because a drop in pressure or resistance is recognized. In order to compensate, the turbine increases its flow. If the flow of 02 is constant the FiO2 falls to 22%. According to the calculation

Formula 1: $Estimated\ FiO2 = \dfrac{(Total\ flow - O2\ flow)*A+(O2*B)}{Total\ flow}$ , again, where A is defined as the percentage of oxygen content in the ambient air surrounding the patient, which can be either set by the user or identified by a sensor in the ventilator, typically this could be a barometric sensor, and where B is defined as the percentage of oxygen in the outlet flow of the supplemental oxygen source which can also be either set by the user or identified by a sensor. In this example the device increases the flow rate of oxygen to maintain the initial Fi02. In this case 4.5 units per minute

FIG. 2B illustrates a second scenario which is indicative of an increased respiratory drive, i.e. the flow volume increasing from X to 1,5*X, the controller determines the new actual FiO2 with the increased flow. The increase in flow consumed by the patient forces the turbine to increase its flow in order to compensate. Again, if the flow of oxygen is constant, the FiO2 falls from 25% to 24%. According to the calculation Formula 1:

$Estimated\ FiO2 = \dfrac{(Total\ flow - O2\ flow)*A+(O2*B)}{Total\ flow}$ the device increases the flow rate of 02 in order to maintain the initial or target FiO2, which in this example is 4.5 units per minute rather than the original 3.

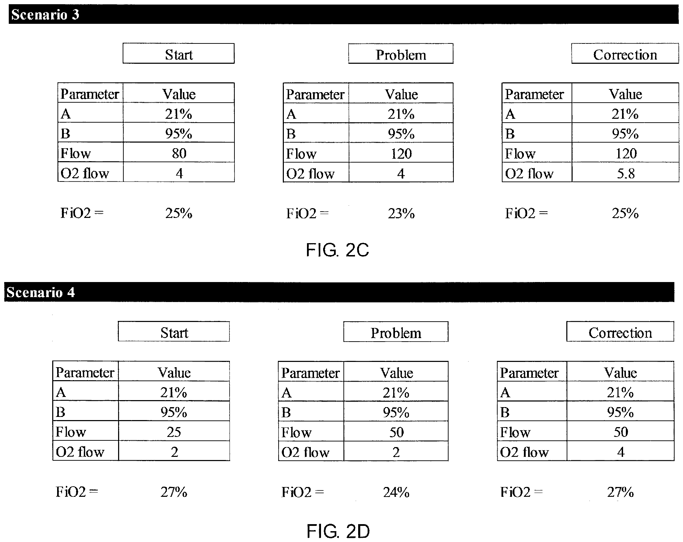

FIG. 2C illustrates a third scenario which is also indicative of a changing respiratory drive, i.e. minute volume, increasing from X to 1,5*X, first the change in the actual values of FiO2 will be calculated or otherwise determined, the increase in minute volume by the patient forces the turbine to increase its flow so as to compensate. In this scenario the flow of oxygen remains the same but the actual Fi02 falls from 25% to 23%.

According to the calculation Formula 1: $Estimated\ FiO2 = \dfrac{(Total\ flow - O2\ flow)*A+(O2*B)}{Total\ flow}$ the device increases the flow rate of O2 to maintain the target FiO2, which in this case is from 4 units per minute to 5.8 units per minute)

FIG. 2D illustrates a fourth scenario which is also indicative of a changing leak levels which could indicate a change of interface, a repositioning during sleep which can cause a varying leak rate due to changes in forces applied on the patient interface, etc. Again, the change to the actual FiO2 will be calculated will be determined based on sensor and flow data. A leakage increase causes a corresponding decrease in the resistances in the patient supply tube being manifested as a pressure decrease, or flow increase, which causes the turbine to increase its flow to so as to maintain the set pressure. Again, if the flow of 02 remains constant the Fi02 falls in this example from 27% to 24%. According to the calculation Formula 1: $Estimated\ FiO2 = \dfrac{(Total\ flow - O2\ flow)*A+(O2*B)}{Total\ flow}$ device increases the flow rate of oxygen to maintain the target FiO2, in this example, from 2 units per minute to 4 units per minute.

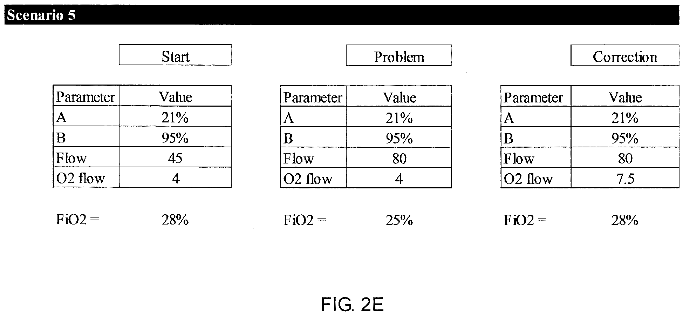

FIG. 2E illustrates a fifth scenario which is indicative of a change of user settings, such as higher IPAP or EPAP needed and set without a manual change of 02 input

Describe what happens, change the values and the FiO2 will be calculated. In this case the user inputs a higher set pressure, the increase in pressures (EPAP and / or IPAP) with constant resistances forces the turbine to increase its flow in order to reach the setpoint pressure which also results in an increase in flow or minute volume, which is actually the more common desired user preference. Therefore, with the corresponding additional increase of the flow of turbine, and the flow of 02 remaining constant, the FiO2 in this example falls from 28% to 25%. The controller then determines the actual Fi02 according to the calculation Formula 1: $Estimated\ FiO2 = \dfrac{(Total\ flow - O2\ flow)*A+(O2*B)}{Total\ flow}$ the device increases the flow rate of oxygen so as to maintain the initial FiO2 of 28%, which thus requires the oxygen flow rate to increase from 4 units per minute to 7.5 units per minute.

While the principles of the invention have been described herein, it is to be understood by those skilled in the art that this description is made only by way of example and not as a limitation as to the scope of the invention. Other embodiments are contemplated within the scope of the present invention in addition to the exemplary embodiments shown and described herein. Modifications and substitutions by one of ordinary skill in the art are considered to be within the scope of the present invention.

## Claims

1. Medical ventilator system configured for use by an individual at home, the ventilator system comprising:

    an oxygen source;
    an air flow generator operatively connected to receive an amount of oxygen from the oxygen source, the air flow generator also being configured to receive an amount of ambient air;

an oxygen control valve being provided between the oxygen source and the air flow generator;

a patient supply tube being configured to receive the amount of ambient air and the amount of oxygen from the air flow generator at a pressure above ambient pressure and deliver the amount of oxygen and the amount of oxygen to a patient interface, the patient interface being configured to interface with one or more breathing airways of a patient;

a pressure sensor provided between the patient and the air flow generator;

a flow sensor provided between the oxygen source and the air flow generator;

a controller, the controller being configured to receive input from the pressure sensor and the flow sensors;

wherein the airflow generator combines the amount of oxygen and the amount of ambient air to form breathing air, wherein the breathing air is supplied to the patient at a pressure above atmospheric pressure; and

wherein the controller is operable to control a flow rate and pressure of the breathing air from the air flow generator and wherein the controller is operable to control an oxygen flow rate to the air flow generator from the oxygen source by selectively opening, closing, or throttling the oxygen control valve.

2. The medical ventilator system of claim 1, further comprising an input module for receiving an input related to a fraction of inspired oxygen (FiO2) setting.

3. The medical ventilator system of claim 2, wherein the controller compares the FiO2 input with a calculated FiO2 value that is based on input data from both the pressure sensor and flow sensor.

4. A method of providing supplemental respiration to a patient in a home environment, the method comprising:

providing an oxygen source;

providing an air flow generator operatively connected to receive an amount of oxygen from the oxygen source, the air flow generator also being configured to receive an amount of ambient air;

providing an oxygen control valve being provided between the oxygen source and the air flow generator;

providing a patient supply tube being configured to receive the amount of oxygen and the amount of ambient air from the air flow generator and deliver the amount of oxygen and the amount of ambient air to a patient interface as breathing air at a pressure above atmospheric pressure;

providing a controller, the controller being configured to receive input from one or more sensors regarding characteristics of the breathing air;

determining an actual fraction of inspired oxygen (FiO2) value within the breathing air being supplied to the patient;

connecting the patient supply tube in an operative manner to one or more airways of the patient;

receiving a target pressure by the controller;

receiving a target fraction of inspired oxygen (Fi02) value for the breathing air by the controller;

increasing or decreasing the output of the air flow generator so as to maintain the target pressure;

maintaining the actual fraction of oxygen in the breathing air at the target fraction of inspired oxygen (Fi02) value by controlling an input of oxygen being supplied from the oxygen source.

5. The method of providing supplemental respiration to a patient of claim 4, wherein the input from the oxygen source is controlled by opening or throttling the oxygen control valve.

6. The method of providing supplemental respiration to a patient of claim 4, wherein the input from the oxygen source is controlled by directly controlling the oxygen source.

7. The method of providing supplemental respiration to a patient of claim 4, further comprising:

providing an oxygen flow sensor between the oxygen source and the air flow generator; and

wherein the determination of the actual fraction of inspired oxygen (FiO2) value is determined based on input received by the controller from the oxygen flow sensor.

8. The method of providing supplemental respiration to a patient of claim 4; further comprising:

providing a breathing air flow sensor on the patient supply tube;

wherein the determination of the actual fraction of inspired oxygen is determined based on input received by the controller from the breathing air flow sensor.

9. The method of providing supplemental respiration to a patient of claim 4, wherein the air flow generator includes a variable speed turbine, and wherein an output flow rate of the air flow generator is determined based on a sensed turbine speed.

10. The method of providing supplemental respiration to a patient of claim 4, further comprising:

recognizing a pressure change in the patient supply tube;
varying an output of the air flow generator so as to compensate for the pressure change;
varying an oxygen flow from the oxygen source to compensate for the increased output of the air flow generator so as to maintain the target fraction of inspired oxygen.

11. A method of providing supplemental respiration to a patient in a home environment, the method comprising providing an oxygen source;
providing an air flow generator operatively connected to receive an amount of oxygen from the oxygen source, the air flow generator also being configured to receive an amount of ambient air;
providing an oxygen control valve being provided between the oxygen source and the air flow generator;
providing a patient supply tube being configured to receive the amount of oxygen and the amount of air from the air flow generator and deliver the amount of oxygen and the amount of air to a patient interface;
providing a controller, the controller being configured to receive input from one or more sensors regarding characteristics of the breathing air;
wherein the airflow generator combines the amount of oxygen and the amount of ambient air to form a breathing air, wherein the breathing air is supplied to the patient at a pressure above atmospheric pressure;
determining an actual fraction of inspired oxygen within the breathing air being supplied to the patient;
connecting the patient supply tube in an operative manner to one or more airways of the patient;
receiving a target pressure by the controller;
receiving a target fraction of inspired oxygen value for the breathing air by the controller;
opening or throttling the oxygen control valve so as to maintain the actual fraction of oxygen in the breathing air at the target fraction of inspired oxygen;
recognizing a pressure change in pressure in the patient supply tube;
varying an output of the air flow generator so as to compensate for the pressure change;
varying an oxygen flow from the oxygen source to compensate for the increased output of the air flow generator so as to maintain the target fraction of inspired oxygen;
providing an oxygen sensor on the patient supply tube;
providing a pressure sensor on the patient supply tube; and
wherein the determination of the actual fraction of inspired oxygen is based on input received from by the controller from the oxygen sensor and the pressure sensor.

12. A method for automatically adjusting oxygenated air for medicinal use comprising the steps of:

combining ambient air and oxygen from an oxygen source by an airflow generator to form breathing air;
sending breathing air through a conduit from the airflow generator at a pressure above ambient to a user interface;
sensing the flow of the breathing air in the conduit between the airflow generator and the user interface;
sensing the flow of oxygen from the oxygen source prior to the oxygen being combined with the ambient air;
determining an estimated fraction of inspired oxygen (FiO2) value by a controller based on input received from sensing both the flow of oxygen and flow of breathing air;
comparing the estimated FiO2 value with a target FiO2 value; and
adjusting an oxygen flow rate of the oxygen source for oxygen received by the airflow generator.

13. The method for automatically adjusting oxygenated air for medicinal use of claim 12, wherein determining the estimated FiO2 value is based on the formula:

$$Estimated\ FiO2 = \frac{(Total\ flow - O2\ flow) * A + (O2 * B)}{Total\ flow}$$

Or

$$O2\ Flow = \frac{(Total\ flow * (TargetFiO2 - A))}{(B - A)}$$

**14.** The method for automatically adjusting oxygenated air for medicinal use of claim 12, further comprising the steps of:

measuring the pressure of breathing air prior to the breathing air being received by the user interface;
comparing the measured breathing air with a target breathing air pressure value; and
adjusting the airflow generator based on the comparison.

**15.** The method for automatically adjusting oxygenated air for medicinal use of claim 12, wherein the determining the estimated FiO2 value step is performed after the adjusting of the airflow generator.

FIG. 1

## Scenario 1

| Start | |
|---|---|

| Parameter | Value |
|---|---|
| A | 21% |
| B | 95% |
| Flow | 50 |
| O2 flow | 2 |

FiO2 =        24%

| Problem | |
|---|---|

| Parameter | Value |
|---|---|
| A | 21% |
| B | 95% |
| Flow | 100 |
| O2 flow | 2 |

FiO2 =        22%

| Correction | |
|---|---|

| Parameter | Value |
|---|---|
| A | 21% |
| B | 95% |
| Flow | 100 |
| O2 flow | 4 |

FiO2 =        24%

FIG. 2A

## Scenario 2

| Start | |
|---|---|

| Parameter | Value |
|---|---|
| A | 21% |
| B | 95% |
| Flow | 50 |
| O2 flow | 3 |

FiO2 =        25%

| Problem | |
|---|---|

| Parameter | Value |
|---|---|
| A | 21% |
| B | 95% |
| Flow | 75 |
| O2 flow | 3 |

FiO2 =        24%

| Correction | |
|---|---|

| Parameter | Value |
|---|---|
| A | 21% |
| B | 95% |
| Flow | 75 |
| O2 flow | 4.5 |

FiO2 =        25%

FIG. 2B

**Scenario 3**

| Start | |
| --- | --- |
| Parameter | Value |
| A | 21% |
| B | 95% |
| Flow | 80 |
| O2 flow | 4 |

FiO2 =      25%

| Problem | |
| --- | --- |
| Parameter | Value |
| A | 21% |
| B | 95% |
| Flow | 120 |
| O2 flow | 4 |

FiO2 =      23%

| Correction | |
| --- | --- |
| Parameter | Value |
| A | 21% |
| B | 95% |
| Flow | 120 |
| O2 flow | 5.8 |

FiO2 =      25%

FIG. 2C

**Scenario 4**

| Start | |
| --- | --- |
| Parameter | Value |
| A | 21% |
| B | 95% |
| Flow | 25 |
| O2 flow | 2 |

FiO2 =      27%

| Problem | |
| --- | --- |
| Parameter | Value |
| A | 21% |
| B | 95% |
| Flow | 50 |
| O2 flow | 2 |

FiO2 =      24%

| Correction | |
| --- | --- |
| Parameter | Value |
| A | 21% |
| B | 95% |
| Flow | 50 |
| O2 flow | 4 |

FiO2 =      27%

FIG. 2D

## Scenario 5

| Start | |
| --- | --- |

| Parameter | Value |
| --- | --- |
| A | 21% |
| B | 95% |
| Flow | 45 |
| O2 flow | 4 |

FiO2 =          28%

| Problem | |
| --- | --- |

| Parameter | Value |
| --- | --- |
| A | 21% |
| B | 95% |
| Flow | 80 |
| O2 flow | 4 |

FiO2 =          25%

| Correction | |
| --- | --- |

| Parameter | Value |
| --- | --- |
| A | 21% |
| B | 95% |
| Flow | 80 |
| O2 flow | 7.5 |

FiO2 =          28%

FIG. 2E

Europäisches
Patentamt

European
Patent Office

Office européen
des brevets

# EUROPEAN SEARCH REPORT

Application Number

EP 17 29 0059

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 040 096 A1 (AIR LIQUIDE MEDICAL SYSTEMS [FR]) 6 July 2016 (2016-07-06) | 1-3 | INV. A61M16/00 A61M16/12 |
| A | * figure 1 * * paragraph [0015] - paragraph [0034] * | 4-15 | |
| X | US 2016/287824 A1 (CHANG SAMUEL M [US]) 6 October 2016 (2016-10-06) | 1-3 | |
| A | * figure 2 * * paragraph [0253] - paragraph [0259] * | 4-15 | |
| X | WO 2016/157106 A1 (FISHER & PAYKEL HEALTHCARE LTD [NZ]) 6 October 2016 (2016-10-06) | 1-3 | |
| A | * figure 1 * * paragraph [00125] - paragraph [00137] * | 4-15 | |
| X | WO 2010/109364 A1 (KONINKL PHILIPS ELECTRONICS NV [NL]; AHMAD SAMIR [US]) 30 September 2010 (2010-09-30) | 1-3 | |
| A | * figure 1 * * page 3, line 28 - page 5, line 29 * | 4-15 | |

TECHNICAL FIELDS
SEARCHED (IPC)

A61M

The present search report has been drawn up for all claims

| Place of search | Date of completion of the search | Examiner |
|---|---|---|
| Munich | 4 August 2017 | Cecchini, Stefano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 29 0059

This annex lists the patent family members relating to the patent documents cited in the above-mentioned European search report.
The members are as contained in the European Patent Office EDP file on
The European Patent Office is in no way liable for these particulars which are merely given for the purpose of information.

04-08-2017

| Patent document cited in search report | | Publication date | Patent family member(s) | | Publication date |
|---|---|---|---|---|---|
| EP 3040096 | A1 | 06-07-2016 | AU | 2015275344 A1 | 21-07-2016 |
| | | | CA | 2916079 A1 | 05-07-2016 |
| | | | CN | 105749392 A | 13-07-2016 |
| | | | EP | 3040096 A1 | 06-07-2016 |
| | | | FR | 3031313 A1 | 08-07-2016 |
| US 2016287824 | A1 | 06-10-2016 | NONE | | |
| WO 2016157106 | A1 | 06-10-2016 | NONE | | |
| WO 2010109364 | A1 | 30-09-2010 | CN | 102361661 A | 22-02-2012 |
| | | | EP | 2411078 A1 | 01-02-2012 |
| | | | JP | 5771182 B2 | 26-08-2015 |
| | | | JP | 2012521252 A | 13-09-2012 |
| | | | RU | 2011142797 A | 27-04-2013 |
| | | | US | 2012006326 A1 | 12-01-2012 |
| | | | WO | 2010109364 A1 | 30-09-2010 |

EPO FORM P0459

For more details about this annex : see Official Journal of the European Patent Office, No. 12/82